# EUROPÄISCHE PATENTANMELDUNG

(11) **EP 2 030 630 A1**
(43) Veröffentlichungstag der Anmeldung: **04.03.2009**
(21) Anmeldenummer: 08105027.0
(22) Anmeldetag: 12.08.2008
(51) Int. Cl.: A61K 41/00, A61K 49/08

(54) **Verfahren zur Abbildung eines Prostatatumors und dafür geeignetes Kontrastmittel**

(30) Priorität: 03.09.2007 DE 102007041836
(71) Anmelder: Siemens Aktiengesellschaft, 80333 München (DE)
(72) Erfinder: Fehre, Jens, 91353 Hausen (DE); Nanke, Ralf, Dr., 91077 Neunkirchen am Brand (DE); Stetter, Martin, Dr., 80997 München (DE)

(57) **Zusammenfassung**

Die Erfindung betrifft ein Verfahren zur Abbildung eines Prostatatumors (1), bei dem dem Patienten ein über die Blutbahn verteilbares Kontrastmittel zugeführt wird, das wenigstens einen Typ von Biomarkern (4) enthält, welche jeweils an ein ferromagnetisches Partikel (8) gebunden sind und an ein für den Tumor typisches, innerhalb des Tumors oder in einem dem Tumor benachbarten Gewebe ausgebildetes Zielmolekül (5) spezifisch binden, wobei die Abbildung mit Hilfe der Magnetresonanz-Tomographie (MRT) erfolgt. Die Erfindung betrifft außerdem ein Kontrastmittel der genannten Art.

## Beschreibung

Die Erfindung betrifft ein Verfahren zur Abbildung eines Prostatatumors und dafür geeignetes Kontrastmittel, wobei sie sich mit dem Problem befasst, einen Prostatatumor möglichst eindeutig, insbesondere bereits noch in einem Vorstadium, zu erkennen bzw. zu diagnostizieren. Das Standardverfahren zur Diagnostik von Prostatakrebs besteht in der Konzentrationsmessung des prostataspezifischen Antigens (PSA) und in einer Tastuntersuchung sowie bei Verdacht in einer Durchführung von Biopsien zur Entnahme von Gewebeproben. Eine derartige Vorgehensweise ist wenig selektiv und kann Prostatakrebs nicht gut von Prostatitis oder BPH unterscheiden. Dies erfordert unter Umständen mehrere für den Patienten unangenehme Biopsien. Neben den genannten Standardverfahren kommen eine Reihe alternativer Verfahren zur Anwendung. Mit der kontrastverstärkten Ultraschalluntersuchung, gegebenenfalls zur Führung von Biopsien, lassen sich Stellen hoher Blutgefäßdichte identifizieren. Eine erhöhte Blutgefäßdichte ist aber nicht für den Prostatatumor spezifisch, da sie auch im Fall der Prostatitis auftritt. Zur Abbildung von Prostatatumoren oder Krebstumoren allgemein kommt eine molekular zielgeführte kontrastverstärkte Ultraschalluntersuchung in Frage. Diese ist zwar kostengünstig, ist aber nicht hochauflösend, so dass sie für hochwertige Diagnosezwecke nur wenig geeignet ist. Schließlich ist noch die optische Bildgebung intrinsischer Signale wie z.B. der Wasserkonzentration eines Gewebes zu nennen.

Aufgabe der Erfindung ist es, ein weiteres Verfahren zur Abbildung eines Prostatatumors und ein dafür geeignetes Kontrastmittel vorzuschlagen.

Diese Aufgabe wird durch ein Verfahren nach Anspruch 1 und ein Kontrastmittel nach Anspruch 8 gelöst. Bei einem erfindungsgemäßen Verfahren wird dem Patienten ein über die Blutbahn verteilbares Kontrastmittel zugeführt wird, das wenigstens einen Typ von Biomarkern enthält, welche jeweils an ein ferromagnetisches Partikel gebunden sind und welche an ein für den Tumor typisches, innerhalb des Tumors oder in einem diesem benachbarten Gewebe ausgebildeten Zielmolekül spezifisch binden. Das interessierende Körpergebiet des Patienten, also die Prostata bzw. ein darin vorhandener Tumor, wird mit Hilfe der Magnetresonanz-Tomographie abgebildet. Auf Grund der spezifisch im Tumorgewebe oder an diesem benachbarten Gewebe spezifisch bindenden Biomarker wird das Kontrastmittel nahezu ausschließlich, zumindest jedoch in überwiegendem Maße in dem Gebiet bzw. in dem Gewebe angereichert, das einer diagnostischen Begutachtung unterzogen werden soll. Die ferromagnetischen, beispielsweise superparamagnetisches Eisenoxid enthaltenden Partikel werden somit gezielt in dem interessierenden Körpergewebe, also einem Prostatatumor angesammelt. Es wird somit gezielt eine Kontrastverstärkung im Wesentlichen nur im Tumorgewebe erreicht, so dass dieser deutlich zu dem ihm umgebenden gesunden Gewebe abgegrenzt ist.

Bei einer bevorzugten Verfahrensvariante werden Biomarker eingesetzt, die an ein für ein bestimmtes Stadium eines Prostatatumors typisches Zielmolekül binden. Auf diese Weise lässt sich der Prostatatumor nicht nur abbilden, sondern es lässt sich auch eine Aussage darüber treffen, in welchem Stadium er sich gerade befindet. Im Stadium beispielsweise der hochgradigen prostatischen intraepithelialen Neoplasie (hgPIN), einem Vorstadium des Prostatakrebses, werden CEACAM-1-Moleküle am Endothel von Blutgefäßen eines dem Prostatatumor benachbarten Gewebes exprimiert. Für das erfindungsgemäße Kontrastmittel wird dementsprechend ein an dieses Molekül bindender Biomarker verwendet, vor allen Dingen ein CEACAM-1-Antikörper. Daneben können auch sogenannte Aptamere bzw. Spiegelmere verwendet werden. Es handelt sich dabei um kurze, stabile und spezifisch bindende RNA-Ketten, wobei die Spiegelmere die spiegelsymmetrischen Pendants der Aptamere sind. Geeignet sind ebenfalls Anticaline, das sind leicht herzustellende Polypeptidketten aus ca. 180 Aminosäuren. Anticaline haben ähnlich spezifische Bindungseigenschaften wie die Antikörper, sind aber leichter herstellbar als diese.

Bei einer weiteren Verfahrensvariante wird auf ein späteres, bereits malignes Stadium des Prostatatumors abgezielt, nämlich auf ein solches, bei dem aufgrund einer stattfindenden Blutgefäßbildung sogenannte Blutgefäßwachstumsfaktoren, z.B. das Molekül VEGF oder Alpha(v)-beta(3)-Integrin im Endothel der Blutgefäße des Tumors zu finden sind. Als Biomarker können ebenfalls die oben genannten Moleküle eingesetzt werden. Daneben kommen auch VEGF- oder Integrin-Liganden Frage. Sowohl bei der auf das hgPIN-Vorstadium als auch auf das Spätstadium des Prostatatumors abzielende Verfahren lassen sich als Biomarker auch für den Menschen unschädliche Viren einsetzen, mit deren spezifisch an die genannten Zielmoleküle bindenden Proteinhülle ein mit dem jeweiligen bildgebenden Verfahren selektierbares Partikel oder Molekül verbunden ist. Bei den Viren kann es sich beispielsweise um M13-Phagen handeln. Die spezifische Bindungseigenschaft der Hüllenproteine der Viren kann etwa durch eine gezielte biologische Evolution erreicht werden, bei der Viren und Zielmoleküle in wässriger Lösung in Kontakt gebracht werden und eine Selektion bindender Viren erfolgt. Hinsichtlich eines erfindungsgemäßen Kontrastmittels gelten die obigen Ausführungen analog.

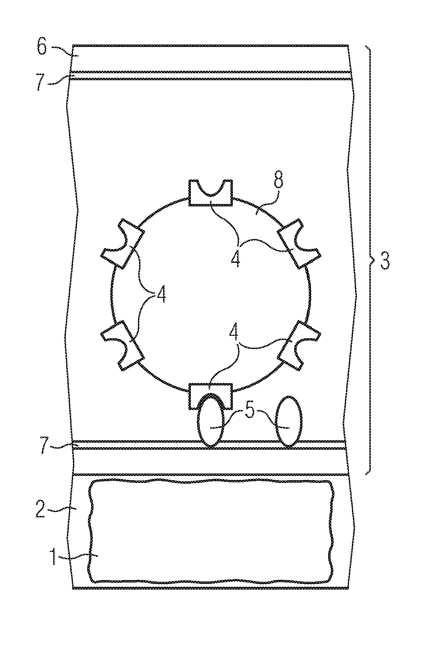

Die beigefügte Abbildung ist eine schematisierte Prinzipdarstellung einer einen Tumor 1 aufweisenden Prostata 2 sowie eines Blutgefäß 3. Blutgefäße 3 sind entweder Bestandteil des Prostatatumors 1, wenn sich dieser bereits im Zustand der Angionese befindet, oder sie sind - im Falle eines Vorstadium des Prostatakrebses, Teile eines diesem benachbarten Gewebes. In jedem Falle sind die vom Kontrastmittel bzw. den darin enthaltenden Biomarkern 4 adressierbaren Zielmoleküle 5 in der Wand 6 bzw. dem Endothel 7 der Blutgefäße 3 vorhanden. Ein Biomarker ist an ein ferromagnetisches, ein paramagnetisches Material wie Eisenoxid enthaltendes Partikel 8 gebunden. Prinzipiell ist es ausreichend, wenn jeweils ein Biomarker 4 mit einem Partikel 8 verbunden ist. Die Partikel 8 haben beispielsweise einen Durchmesser von 10nm - 100nm. Um eine zuverlässige Anbindung an die Zielmoleküle 5 zu erreichen ist es vorteilhaft, wenn mehrere Biomarker 4 mit dem Partikel 8 verbunden sind, wenn dieses quasi mit Biomarkern 4 beschichtet ist. Dadurch ist gewährleistet, dass in einer Vielzahl unterschiedlicher Drehstellungen des Partikels 8 eine Bindung an ein Zielmolekül 5 erfolgen kann. Nach der beispielsweise intravenös erfolgten Verabreichung des Kontrastmittels folgt schon bereits nach kurzer Zeit eine Anreicherung im Zielgewebe also im Prostatatumor 1, so dass dann eine Aufnahme mit MRT erfolgen kann. Wenn beispielsweise nicht fest steht oder nicht sicher ist, ob ein Tumor in einem Früh- oder Spätstadium vorliegt, kann das Kontrastmittel sowohl Biomarker enthalten, die an Zielmoleküle des Frühstadiums (CEACAM-1) oder des späteren Stadiums (etwa VEGF oder Alpha(v)-beta(3)-Integrin binden.

## Patentansprüche

1. Verfahren zur Abbildung eines Prostatatumors (1), bei dem dem Patienten ein über die Blutbahn verteilbares Kontrastmittel zugeführt wird, das wenigstens einen Typ von Biomarkern (4) enthält, welche jeweils an ein ferromagnetisches Partikel (8) gebunden sind und an ein für den Tumor typisches, innerhalb des Tumors oder in einem dem Tumor benachbarten Gewebe ausgebildetes Zielmolekül (5) spezifisch binden, wobei die Abbildung mit Hilfe der Magnetresonanz-Tomographie (MRT) erfolgt.

2. Verfahren nach Anspruch 1, bei dem ein Kontrastmittel mit Biomarkern (4) eingesetzt wird, die an ein für ein bestimmtes Stadium eines Prostatatumors typisches Zielmolekül (5) binden.

3. Verfahren nach Anspruch 2, bei dem an das CEACAM-1-Molekül bindende Biomarker (4) verwendet werden.

4. Verfahren nach Anspruch 3, bei dem Biomarker aus der Gruppe "Antikörper, Aptamer, Spiegelmer, Anticalin, Virus" verwendet werden.

5. Verfahren nach Anspruch 2, bei dem an VEGF bindende Biomarker (4) verwendet werden.

6. Verfahren nach Anspruch 2, bei dem an Alpha(v)-beta(3)-Integrin bindende Biomarker (4) verwendet werden.

7. Verfahren nach Anspruch 5 oder 6, bei dem ein Biomarker (4) aus der Gruppe "Antikörper, Ligand, Aptamer, Spiegelmer, Anticalin, Virus" verwendet wird.

8. Kontrastmittel zur Abbildung eines Prostatatumors (1) mit Hilfe der Magnetresonanz-Tomographie, enthaltend wenigstens einen Typ von Biomarkern (4), die an ein für den Tumor typisches, innerhalb des Tumors oder in dem Tumor benachbarten Gewebe ausgebildetes Zielmolekül spezifisch binden, wobei die Biomarker jeweils an ferromagnetisches Partikel (8) gebunden sind.

9. Kontrastmittel nach Anspruch 8, mit Biomarkern (4), die an ein für ein bestimmtes Stadium eines Prostatatumors typisches Zielmolekül (5) binden.

10. Kontrastmittel nach Anspruch 9, mit an das CEACAM-1-Molekül bindenden Biomarkern (4).

11. Kontrastmittel nach Anspruch 10, mit einem Biomarker aus der Gruppe "Antikörper, Aptamer, Spiegelmer, Anticalin, Virus".

12. Kontrastmittel nach Anspruch 9, mit an VEGF bindenden Biomarkern (4).

13. Kontrastmittel nach Anspruch 9, mit einem an Alpha(v)-beta(3)-Integrin bindenden Biomarker (4).

14. Kontrastmittel nach Anspruch 12 oder 13, mit einem Biomarker (4) aus der Gruppe "Antikörper, Ligand, Aptamer, Spiegelmer, Anticalin, Virus".
